(19) Europäisches Patentamt
European Patent Office
Office européen des brevets

(11) EP 1 166 749 B1

(12) # EUROPÄISCHE PATENTSCHRIFT

(45) Veröffentlichungstag und Bekanntmachung des Hinweises auf die Patenterteilung:
**12.10.2005 Patentblatt 2005/41**

(51) Int Cl.7: **A61K 7/13**

(21) Anmeldenummer: **01106293.2**

(22) Anmeldetag: **15.03.2001**

(54) **Mittel und Verfahren zur Färbung keratinischer Fasern**

Composition and process for dyeing keratinic fibres

Composition et procédé pour la teinture des fibres kératiniques

(84) Benannte Vertragsstaaten:
**AT BE CH CY DE DK ES FI FR GB GR IE IT LI LU MC NL PT SE TR**

(30) Priorität: **01.07.2000 DE 10032135**

(43) Veröffentlichungstag der Anmeldung:
**02.01.2002 Patentblatt 2002/01**

(73) Patentinhaber: **Wella Aktiengesellschaft
64274 Darmstadt (DE)**

(72) Erfinder:
- **Braun, Hans-Jürgen, Dr.
3182 Überstorf (CH)**
- **Umbricht, Gisela, Dr.
1723 Marly (CH)**

(56) Entgegenhaltungen:
**AT-B- 363 604**
**DE-U- 20 000 268**
**DE-A- 19 643 059**
**US-A- 5 518 507**

EP 1 166 749 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft eine Zusammensetzung zur Färbung keratinischer Fasern, insbesondere menschlicher Haare, mit einem Gehalt an 2,4-Diamino-1-(2-methoxyethoxy)-benzol und mindestens einem p-Phenylendiaminderivat, 4,5-Diamino-1H-pyrazolderivat oder p-Aminophenolderivat sowie ein Verfahren zur Färbung keratinischer Fasern unter Verwendung dieser Zusammensetzung.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels.

An Oxidationsfarbstoffe, die zur Färbung menschlicher Haare verwendet werden sollen, sind zahlreiche besondere Anforderungen gestellt. Die Farbstoffe müssen in toxikologischer und dermatologischer Hinsicht unbedenklich sowie nicht sensibilisierend sein und Färbungen in der gewünschten Intensität ermöglichen. Die mit Oxidationsfarbstoffen erhaltenen Haarfärbungen sollen für mindestens vier bis sechs Wochen stabil bleiben. Es wird auch erwartet, dass die Haarfärbungen bei der Anwendung von weiteren kosmetischen Behandlungen, wie beispielsweise Shampoonieren oder Haarumformung, stabil bleiben. Weiterhin sollen diese Haarfärbungen auch gegen äußere Einwirkungen, zum Beispiel Licht, Wetter, Schweiß oder mechanischem Abrieb, stabil sein.
Außerdem ist es erforderlich, dass durch Kombination geeigneter Entwicklersubstanzen und Kupplersubstanzen eine breite Palette verschiedener Farbnuancen erzeugt werden kann.

Ein besonderes Problem stellt die Erzeugung von stabilen blauen und rötlichen Farbtönen dar. Blaufärbende Haarfarbkuppler werden zusammen mit gelben und roten Farbkomponenten zur Erzeugung von natürlich wirkenden Haarfärbungen benötigt. Modische rötliche Haarfärbungen werden dagegen durch Verwendung eines Überschusses an roten Farbkomponenten erzeugt. Die Stabilität der Haarfärbungen sowohl im Blaubereich als auch im Rotbereich lässt bei herkömmlichen Haarfärbemitteln besonders hinsichtlich der Einwirkung von organischen Säuren beziehungsweise Schweiß jedoch noch viele Wünsche offen.

Die Darstellung von 2,4-Diamino-1-(2-methoxyethoxy)benzol sowie dessen Verwendung als Kupplersubstanz in Oxidationshaarfärbemitteln ist aus der Literatur, zum Beispiel der US-PS 4 125 367, US-PS 4 259 261 und US-PS 4 329 504, bekannt.

Es wurde nun gefunden, dass mit einer Kombination bestehend aus der Kupplersubstanz 2,4-Diamino-1-(2-methoxyethoxy)benzol und mindestens einer Entwicklersubstanz aus der Gruppe bestehend aus bestimmten p-Phenylendiaminderivaten, 4,5-Diamino-1H-pyrazolderivaten und p-Aminophenolderivaten Färbemittel für Keratinfasern erhalten werden, die neben einer hervorragenden Waschstabilität und Lichtstabilität eine gegenüber dem Stand der Technik wesentlich verbesserte Stabilität gegenüber der Einwirkung von organischen Säuren beziehungsweise Schweiß aufweisen.

Gegenstand der vorliegenden Erfindung ist daher ein Mittel zur Färbung von Keratinfasern, insbesondere menschlichen Haaren, auf der Basis einer Entwicklersubstanz-Kupplersubstanz-Kombination, welches dadurch gekennzeichnet ist, dass es die Kupplersubstanz 2,4-Diamino-1-(2-methoxyethoxy)benzol oder deren physiologisch verträgliches Salz und mindestens eine Entwicklersubstanz aus der Gruppe bestehend aus

(a) am Benzolring monosubstituierten p-Phenylendiaminderivaten der allgemeinen Formel (I) oder deren physiologisch verträglichen Salzen,

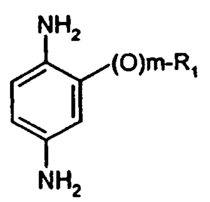 (I) ,

worin wenn **m** gleich 0 ist $\mathbf{R_1}$ einen geradkettigen oder verzweigten Monohydroxy-($C_1$-$C_6$)-alkylrest, einen geradkettigen oder verzweigten Polyhydroxy-($C_2$-$C_6$)-alkylrest, einen geradkettigen oder verzweigten Mono-($C_1$-$C_6$)-alkoxy-($C_1$-$C_6$)-alkylrest, einen geradkettigen oder verzweigten Poly-($C_1$-$C_6$)-alkoxy-($C_2$-$C_6$)-alkylrest, einen geradkettigen oder verzweigten Amino-($C_1$-$C_6$)-Alkylrest, oder eine carbozyklische oder heterozyklische, substituierte oder unsubstituierte, aromatische Verbindung darstellt; und wenn **m** gleich 1 ist $\mathbf{R_1}$ einen geradkettigen oder verzweigten $C_3$-$C_6$-Alkylrest, einen geradkettigen oder verzweigten Monohydroxy-($C_2$-$C_6$)-alkylrest, einen geradkettigen oder verzweigten Polyhydroxy-($C_3$-$C_6$)-alkylrest, einen geradkettigen oder verzweigten Mono($C_1$-$C_6$)-alkoxy-($C_2$-$C_6$)-alkylrest, einen geradkettigen oder verzweigten Poly-($C_1$-$C_6$)-alkoxy-($C_3$-$C_6$)-alkylrest, einen geradkettigen oder verzweigten Amino-($C_2$-$C_6$)-Alkylrest, oder einen carbozyklische oder heterozyklische, substituierte

oder unsubstituierte, aromatische Verbindung darstellt;

(b) 4,5-Diamino-1H-pyrazolderivaten der allgemeinen Formel (II) oder deren physiologisch verträglichen Salzen,

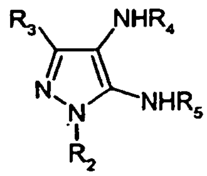

(II)

,

worin $\mathbf{R_2}$, $\mathbf{R_4}$ und $\mathbf{R_5}$ unabhängig voneinander ein Wasserstoffatom, einen geradkettigen oder verzweigten $C_1$-$C_6$-Alkylrest, einen geradkettigen oder verzweigten Monohydroxy-($C_1$-$C_6$)-alkylrest, einen geradkettigen oder verzweigten Polyhydroxy-($C_2$-$C_6$)-alkylrest, oder einen unsubstituierten oder am Aromaten substituierten Benzylrest darstellen,

und $\mathbf{R_3}$ gleich einem Wasserstoffatom, einem geradkettigen oder verzweigten $C_1$-$C_6$-Alkylrest, einem geradkettigen oder verzweigten Monohydroxy-($C_1$-$C_6$)-alkylrest, einem geradkettigen oder verzweigten Polyhydroxy-($C_2$-$C_6$)-alkylrest, einem geradkettigen oder verzweigten Mono($C_1$-$C_6$)-alkoxy-($C_1$-$C_6$)-alkylrest, einem geradkettigen oder verzweigten Poly-($C_1$-$C_6$)-alkoxy-($C_2$-$C_6$)-alkylrest, oder einer carbozyklischen oder heterozyklischen, substituierten oder unsubstituierten, aromatischen Verbindung ist; und

(c) 4-Aminophenolderivaten der allgemeinen Formel (III) oder deren physiologisch verträglichen Salzen,

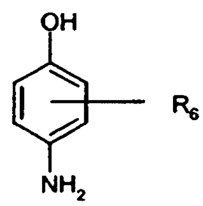

(III)

,

worin $\mathbf{R_6}$ gleich einem geradkettigen oder verzweigten $C_1$-$C_6$-Alkylrest, einem geradkettigen oder verzweigten Monohydroxy-($C_1$-$C_6$)-alkylrest, einem geradkettigen oder verzweigten Polyhydroxy-($C_2$-$C_6$)-alkylrest, einem geradkettigen oder verzweigten Mono-($C_1$-$C_6$)-alkoxy-($C_1$-$C_6$)-alkylrest, einem geradkettigen oder verzweigten Poly-($C_1$-$C_6$)-alkoxy-($C_2$-$C_6$)-alkylrest, einem geradkettigen oder verzweigten Amino-($C_1$-$C_6$)-Alkylrest, oder einer carbozyklischen oder heterozyklischen, substituierten oder unsubstituierten, aromatischen Verbindung ist, unter der Bedingung, dass der Rest $\mathbf{R_6}$ nicht gleich Methyl ist, wenn er in 2-Stellung steht; enthält.

Bevorzugte p-Phenylendiaminderivate der Formel (I) sind 2-(Hydroxymethyl)-p-phenylendiamin, 2-(1-Hydroxyethyl)-p-phenylendiamin, 2-(2-Hydroxyethyl)-p-phenylendiamin, 2-(3-Hydroxypropyl)-p-phenylendiamin, 2-(1,2-Dihydroxyethyl)-p-phenylendiamin, 2-(Methoxymethyl)-p-phenylendiamin, 2-(2-Methoxyethyl)-p-phenylendiamin, 2-(2-Hydroxyethoxy)-p-phenylendiamin, 2,5-Diamino-1,1'-biphenyl, 2-(2-Thienyl)-p-phenylendiamin, 2-(3-Thienyl)-p-phenylendiamin und 3-(2,5-Diaminophenyl)pyridin.

Bevorzugte Pyrazolderivate der Formel (II) sind 4,5-Diamino-1-methyl-1 H-pyrazol, 4,5-Diamino-1-(2-hydroxyethyl)-1H-pyrazol, 4,5-Diamino-1-(1-methylethyl)-1 H-pyrazol, 4,5-Diamino-1-(phenylmethyl)-1 H-pyrazol, 4,5-Diamino-1-((4-methylphenyl)methyl)-1H-pyrazol, 4,5-Diamino-1-(2-hydroxyethyl)-3-methyl-1H-pyrazol, 4,5-Diamino-1-methyl-3-phenyl-1 H-pyrazol und 4,5-Diamino-1,3-dimethyl-1H-pyrazol.

Bevorzugte p-Aminophenolderivate der Formel (III) sind 3-Methyl-4-aminophenol, 2-Aminomethyl-4-aminophenol, 2-Hydroxymethyl-4-aminophenol, 3-Hydroxymethyl-4-aminophenol, 2-Phenyl-4-aminophenol, 2-(2-Thienyl)-4-aminophenol, 2-(3-Thienyl)-4-aminophenol, 3-(2-Thienyl)-4-aminophenol und 3-(3-Thienyl)-4-aminophenol.

Besonders bevorzugt sind Haarfärbemittel, welche eine der folgenden erfindungsgemäßen Entwicklersubstanz-Kupplersubstanz-Kombinationen enthalten:

- 2,4-Diamino-1-(2-methoxyethoxy)benzol und 2-(1-Hydroxyethyl)-p-phenylendiamin;
- 2,4-Diamino-1-(2-methoxyethoxy)benzol und 2-(2-Hydroxyethyl)-p-phenylendiamin;
- 2,4-Diamino-1-(2-methoxyethoxy)benzol und 2-(3-Hydroxypropyl)-p-phenylen-diamin;
- 2,4-Diamino-1-(2-methoxyethoxy)benzol und 2-(1,2-Dihydroxyethyl)-p-phenylendiamin;
- 2,4-Diamino-1-(2-methoxyethoxy)benzol und 2-(Methoxymethyl)-p-phenylen-diamin;

- 2,4-Diamino-1-(2-methoxyethoxy)benzol und 2-(2-Methoxyethyl)-p-phenylen-diamin;
- 2,4-Diamino-1-(2-methoxyethoxy)benzol und 2-(2-Hydroxyethoxy)-p-phenylendiamin;
- 2,4-Diamino-1-(2-methoxyethoxy)benzol und 2,5-Diaminobiphenyl;
- 2,4-Diamino-1-(2-methoxyethoxy)benzol und 2-(2-Thienyl)-p-phenylendiamin;
- 2,4-Diamino-1-(2-methoxyethoxy)benzol und 2-(3-Thienyl)-p-phenylendiamin;
- 2,4-Diamino-1-(2-methoxyethoxy)benzol und 3-(2,5-Diaminophenyl)-pyridin;
- 2,4-Diamino-1-(2-methoxyethoxy)benzol und 4,5-Diamino-1-methyl-1H-pyrazol;
- 2,4-Diamino-1-(2-methoxyethoxy)benzol und 4,5-Diamino-1-(2-hydroxyethyl)-1H-pyrazol;
- 2,4-Diamino-1-(2-methoxyethoxy)benzol und 4,5-Diamino-1-(1-methylethyl)-1H-pyrazol;
- 2,4-Diamino-1-(2-methoxyethoxy)benzol und 4,5-Diamino-1-(phenylmethyl)-1H-pyrazol;
- 2,4-Diamino-1-(2-methoxyethoxy)benzol und 4,5-Diamino-1-((4-methyl-phenyl)methyl)-1H-pyrazol;
- 2,4-Diamino-1-(2-methoxyethoxy)benzol und 4,5-Diamino-1-(2-hydroxyethyl)-3-methyl-1H-pyrazol;
- 2,4-Diamino-1-(2-methoxyethoxy)benzol und 4,5-Diamino-1-methyl-3-phenyl-1H-pyrazol;
- 2,4-Diamino-1-(2-methoxyethoxy)benzol und 3-Methyl-4-aminophenol;
- 2,4-Diamino-1-(2-methoxyethoxy)benzol und 2-Aminomethyl-4-aminophenol;
- 2,4-Diamino-1-(2-methoxyethoxy)benzol und 2-Phenyl-4-aminophenol;
- 2,4-Diamino-1-(2-methoxyethoxy)benzol und 2-(2-Thienyl)-4-aminophenol;
- 2,4-Diamino-1-(2-methoxyethoxy)benzol und 2-(3-Thienyl)-4-aminophenol;
- 2,4-Diamino-1-(2-methoxyethoxy)benzol und 3-(2-Thienyl)-4-aminophenol;
- 2,4-Diamino-1-(2-methoxyethoxy)benzol und 3-(3-Thienyl)-4-aminophenol;
- 2,4-Diamino-1-(2-methoxyethoxy)benzol, 2-(2-Hydroxyethyl)-p-phenylendiamin und 3-Methyl-4-aminophenol;
- 2,4-Diamino-1-(2-methoxyethoxy)benzol, 2-(2-Hydroxyethyl)-p-phenylendiamin und 4,5-Diamino-1-(2-hydroxyethyl)-1H-pyrazol;
- 2,4-Diamino-1-(2-methoxyethoxy)benzol, 2-Methoxymethyl-p-phenylendiamin und 3-Methyl-4-aminophenol;
- 2,4-Diamino-1-(2-methoxyethoxy)benzol, 2-Methoxymethyl-p-phenylendiamin und 4,5-Diamino-1-(2-hydroxyethyl)-1H-pyrazol;
- 2,4-Diamino-1-(2-methoxyethoxy)benzol, 2-(2-Thienyl)-p-phenylendiamin und 3-Methyl-4-aminophenol;
- 2,4-Diamino-1-(2-methoxyethoxy)benzol, 2-(2-Thienyl)-p-phenylendiamin und 4,5-Diamino-1-(2-hydroxyethyl)-1H-pyrazol;
- 2,4-Diamino-1-(2-methoxyethoxy)benzol, 2,5-Diamino-1,1'-biphenyl und 3-Methyl-4-aminophenol;
- 2,4-Diamino-1-(2-methoxyethoxy)benzol, 2,5-Diamino-1,1'-biphenyl und 4,5-Diamino-1-(2-hydroxyethyl)-1H-pyrazol;
- 2,4-Diamino-1-(2-methoxyethoxy)benzol, 2-(2-Hydroxyethoxy)-p-phenylendiamin und 3-Methyl-4-aminophenol;
- 2,4-Diamino-1-(2-methoxyethoxy)benzol, 2-(2-Hydroxyethoxy)-p-phenylendiamin und 4,5-Diamino-1-(2-hydroxyethyl)-1H-pyrazol.

Zur Abrundung des Farbergebnisses sowie zur Erzeugung von speziellen Farbeffekten können den erfindungsgemäßen Kombinationen weitere Entwicklersubstanzen und/oder Kupplersubstanzen sowie direktziehende Farbstoffe zugesetzt werden.

Als Entwicklersubstanzen können beispielsweise die folgenden Verbindungen genannt werden: 1,4-Diaminobenzol (p-Phenylendiamin); 1,4-Diamino-2-methylbenzol; 1,4-Diamino-2,6-dimethylbenzol; 2,5-Diamino-1,3-diethylbenzol, 1,4-Diamino-2,5-dimethylbenzol; 1,4-Diamino-2,3-dimethyl-benzol; 2-Chlor-1,4-diamino-benzol; 1-(Aminomethyl)-2,5-diaminobenzol; 2-(2-(Acetylamino)ethoxy)-1,4-diaminobenzol; 4-Phenylamino-anilin; 4-Dimethylamino-anilin; 4-(Dipropylamino)anilin; 4-Diethylamino-anilin; 4-(Ethyl(2-hydroxyethyl)amino)anilin; 4-(Di(2-hydroxyethyl)amino)-anilin; 4-(Di(2-hydroxyethyl)amino)-2-methylanilin; 4-((2-Methoxyethyl)amino)-anilin; 4-((3-Hydroxypropyl)amino)-anilin; 4-((2,3-Dihydroxypropyl)-amino)anilin; 1,3-Bis-((4-aminophenyl)(2-hydroxyethyl)amino)-2-propanol; 1,4-Di((4-Aminophenyl)amino)butan; 1,8-Bis-(2,5-diaminophenoxy)-3,6-dioxaoctan; 4-Aminophenol; 4-Amino-3-fluorphenol; 4-Methylamino-phenol; 4-Amino-2-(aminomethyl)-phenol; 4-Amino-2-(hydroxymethyl)-phenol; 4-Amino-2-fluorphenol; 4-Amino-2-((2-hydroxyethyl)amino)methyl-phenol; 4-Amino-2-methylphenol; 4-Amino-2-(methoxymethyl)-phenol; 4-Amino-2-(2-hydroxyethyl)-phenol; 5-Amino-salicylsäure; 2,5-Diamino-pyridin; 2,4,5,6-Tetraamino-pyrimidin und 2,5,6-Triamino-4(1H)-pyrimidon.

Als Kupplersubstanzen können beispielsweise die folgenden Verbindungen genannt werden: 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)-amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylaminophenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol (Resorcin), 1-Chlor-2,4-dihydroxy-benzol, 2-Methyl-1,3-resorcin (2-Methylresorcin), 2-Chlor-1,3-dihydroxy-benzol (2-Chlorresorcin), 1,2-Dichlor-3,5-dihydroxy-4-methylbenzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)-amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol, 7-Hydroxy-indol und 2,3-Indolindion.

Weiterhin kann das erfindungsgemäße Färbemittel zusätzlich weitere Farbkomponenten, beispielsweise 6-Amino-2-methylphenol und 2-Amino-5-methylphenol, sowie übliche direktziehende Farbstoffe aus der Gruppe der sauren oder basischen Farbstoffe, Triphenylmethanfarbstoffe, Anthrachinonfarbstoffe, aromatischen Nitrofarbstoffe, Azofarbstoffe, Lebensmittelfarbstoffe oder Dispersionsfarbstoffe, enthalten. Das erfindungsgemäße Färbemittel kann diese Farbkomponenten in einer Menge von etwa 0,01 bis 5 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Das erfindungsgemäße Haarfärbemittel enthält das 2,4-Diamino-1-(2-methoxyethoxy)benzol sowie die Verbindungen der Formel (I) bis (III) jeweils in einer Menge von etwa 0,01 bis 5 Gewichtsprozent, vorzugsweise 0,01 bis 3,0 Gewichtsprozent, wobei die Gesamtmenge an Entwicklersubstanzen und Kupplersubstanzen in dem erfindungsgemäßen Mittel etwa 0,05 bis 20 Gewichtsprozent, vorzugsweise etwa 0,1 bis 10 Gewichtsprozent, beträgt.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung, eine Creme, ein Gel, ein Aerosolschaum oder eine Emulsion sein, wobei die Verwendung in Form einer Creme, eines Geles oder einer Emulsion besonders bevorzugt ist. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von etwa 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure, Zitronensäure oder Weinsäure, in Betracht.

Für die Anwendung zur oxidativen Färbung von Haaren vermischt man das vorstehend beschriebene Färbemittel vor dem Gebrauch mit einem Oxidationsmittel und trägt eine je nach Haarfülle für die Haarfärbehandlung ausreichende Menge, im allgemeinen etwa 60 bis 200 Gramm, dieses Gemisches auf das Haar auf.

Als Oxidationsmittel zur Entwicklung der Haarfärbung kommen insbesondere Wasserstoffperoxid oder dessen Additionsverbindungen an Harnstoff, Melamin, Natriumborat oder Natriumcarbonat in Form einer etwa 3- bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel etwa 5:1 bis 1:2, vorzugsweise jedoch etwa 1:1. Größere Mengen an Oxidationsmittel werden vor allem bei höheren Farbstoffkonzentrationen im Haarfärbe-mittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel zeichnet sich durch eine ausgezeichnete physiologische Verträglichkeit aus und ermöglicht Färbungen mit ausgezeichneter Farbechtheit, insbesondere was die Lichtechtheit, Waschechtheit und Reibeechtheit sowie die Stabilität gegenüber der Einwirkung von Säuren beziehungsweise der Absonderung von Schweiß anbetrifft. Weiterhin hat sich gezeigt, dass insbesondere bei Verwendung des Chloridsalzes des 2,4-Diamino-1-(2-methoxyethoxy)benzols äußerst stabile Färbungen eralten werden. Hinsichtlich der färberischen Eigenschaften bieten die erfindungsgemäßen Färbemittel je nach Art und Zusammensetzung der Farbkomponenten eine breite Palette verschiedener Farbnuancen, die sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der erfindungsgemäßen Färbemittel zeigen sich weiterhin darin, dass diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

**Beispiele**

**Beispiel 1: Herstellung von 2,4-Diamino-1-(2-methoxyethoxy)-benzol-Dihydrochlorid**

1. Stufe: 2,4-Dinitro-1-(2-methoxyethoxy)benzol

50 g (0,25 mol) 1-Chlor-2,4-dinitro-benzol werden in 216 ml Methylglykol mit 10 g (0,25 mol) Natriumhydroxid (in Form einer 10 n Lösung in Methylglykol) langsam versetzt und 1 Stunde bei 100 °C erhitzt. Die Reaktionsmischung wird auf 300 ml Wasser gegossen. Anschließend wird 3mal mit 200 ml Dichlormethan extrahiert und die organische Phase über Natriumsulfat getrocknet, filtriert und eingeengt. Man erhält 53,8 g (89 % der Theorie) eines viskosen, leicht bräunlichen Öles.
$^1$H-NMR (300 MHz, $CDCl_3$): δ = 8,73 ppm (d; J = 2,7 Hz; 1 H); 8,41 ppm (dd; J = 2,7; 9,3 Hz; 1H); 7,27 ppm (d; J = 9,3 Hz; 1H); 4,39 ppm (t; J = 4,5 Hz; 2H); 3,83 ppm (t; J = 4,5 Hz; 2H); 3,44 ppm (s; 3H).

2. Stufe: 2,4-Diamino-1-(2-methoxyethoxy)benzol -Dihydrochlorid

40 g (0,02 mol) 2,4-Dinitro-1-(2-methoxyethoxy)benzol werden in 300 ml Ethanol an 5 g Palladium (10 % auf Aktivkohle) bei 5 bar Wasserstoffdruck hydriert. Nach einem Tag wird der Katalysator unter Inertgas entfernt und die erhaltene Lösung in einer 3 n Salzsäure in Ethanol eingerührt und partiell aufkonzentriert. Absaugen und Trocknen liefert 20,1 g (40 % der Theorie) eines grauen Produkts mit einem Schmelzpunkt von 215 °C.
$^1$H-NMR (DMSO-$D_6$, 300 MHz): δ = 7,34-7,09 ppm (m; 3H); 6,0-4,5 ppm (br; NH); 4,20 ppm (t; J = 4,5 Hz; 2H); 3,37 ppm (t; J = 4,5 Hz; 2H); 3,34 ppm (s; 3H).

| Elementaranalyse: | | | | |
|---|---|---|---|---|
| $C_9H_{16}N_2O_2$•2HCl (255,14) | %C | %H | %N | %Cl |
| ber. | 42,4 | 6,3 | 11,0 | 27,8 |
| gef. | 42,3 | 6,4 | 10,8 | 27,3 |

**Beispiele 2 - 19: Haarfärbelösungen mit einem basischen pH-Wert**

| | |
|---|---|
| 10,00 g | Ethanol |

(fortgesetzt)

| | |
|---|---|
| 10,00 g | Natriumlaurylethersulfat, 28%ige Lösung in Wasser |
| 10,00 g | Ammoniak, 25%ige wässrige Lösung |
| 0,30 g | Ascorbinsäure |
| 0,67 g | 2,4-Diamino-1-(2-methoxyethoxy)benzol-dihydrochlorid |
| X g | Entwicklersubstanz gemäß Tabelle 1 |
| ad 100,00 g | Wasser, entmineralisiert |

Vor der Anwendung werden 10 Gramm der vorstehenden Haarfärbelösung mit 10 Gramm einer 6%igen wässrigen Wasserstoffperoxidlösung gemischt. Das so erhaltene Oxidationshaarfärbemittel, das einen pH-Wert zwischen 9 und 11 aufweist, wird anschließend in der erforderlichen Menge auf gebleichte Haare aufgetragen. Nach einer Einwirkungszeit von 30 Minuten bei 40 °C wird mit einem Farbpflegeshampoo gewaschen, mit Wasser gespült und getrocknet. Die erhaltenen Färbungen und Farbintensitäten sind in Tabelle 1 zusammengefasst.

Tabelle 1:

| **Haarfärbeispiele 2 - 19** | | | |
|---|---|---|---|
| **Beispiel Nr.** | **Entwicklersubstanz X** | **Menge an X in Gramm** | **Farbton** |
| 2 | 2-(Hydroxymethyl)-p-phenylendiamin | 0,35 | dunkelblau |
| 3 | 2-(1-Hydroxyethyl)-p-phenylendiamin | 0,38 | violettstichig blau |
| 4 | 2-(2-Hydroxyethyl)-p-phenylendiaminsulfat | 0,38 | dunkelblau |
| 5 | 2-(1,2-Dihydroxyethyl)-p-phenylendiamin | 0,42 | blau |
| 6 | 2-(Methoxymethyl)-p-phenylendiamin *2HCl | 0,42 | violettstichig blau |
| 7 | 2-(2-Hydroxyethoxy)-p-phenylendiamin | 0,42 | blau-schwarz |
| 8 | 2-(3-Hydroxypropyl-p-phenylendiamin | 0,42 | blau |
| 9 | 2-(2-Thienyl)-p-phenylendiamin*2HCl | 0,48 | grünstichig blau |
| 10 | 4,5-Diamino-1-methyl-1H-pyrazol-sulfat | 0,28 | purpurrot |
| 11 | 4,5-Diamino-1-(2-hydroxyethyl)-1H-pyrazol-sulfat | 0,36 | purpurrot |
| 12 | 4,5-Diamino-1-(1-methylethyl)-1H-pyrazol-sulfat | 0,35 | purpurrot |
| 13 | 4,5-Diamino-1-(phenylmethyl)-1H-pyrazol-sulfat | 0,47 | purpurrot |
| 14 | 4,5-Diamino-1-((4-methylphenyl)methyl)-1 H-pyrazol-sulfat | 0,51 | purpurrot |
| 15 | 4,5-Diamino-1-(2-hydroxyethyl)-3-methyl-1 H-pyrazol*2HCl | 0,39 | violett |
| 16 | 4,5-Diamino-1-methyl-3-phenyl-1H-pyrazol*2HCl | 0,47 | blau violett |
| 17 | 3-Methyl-4-aminophenol | 0,31 | purpurrot |
| 18 | 2-Hydroxymethyl-4-aminophenol | 0,35 | rosérot |
| 19 | 3-Hydroxymethyl-4-aminophenol | 0,35 | rosé |

**Beispiele 20 - 28: Haarfärbecreme, basisch**

| | |
|---|---|
| 15,00 g | Cetylstearylalkohol 50/50 |
| 5,00 g | Glycerinmonostearat |
| 10,00 g | Natriumlaurylethersulfat, 28%ige wässrige Lösung |
| 0,30 g | Natriumsulfit |
| 5,00 g | Ammoniak, 25%ige wässrige Lösung |
| X g | Farbstoff gemäß Tabelle 2 |
| ad 100,00 g | Wasser, entmineralisiert |

Unmittelbar vor der Anwendung wird die vorstehende Färbecreme mit 100 Gramm einer 6%igen wässrigen

Wasserstoffperoxidlösung vermischt. 60 Gramm des so erhaltenen Oxidationshaarfärbemitteln werden anschließend auf mittelblonde Naturhaare aufgetragen. Nach einer Einwirkungszeit von 30 Minuten bei 40 °C wird das Haar mit lauwarmem Wasser gespült und sodann getrocknet.

Die Färbeergebnisse sind in Tabelle 3 zusammengefasst.

**Tabelle 2: Haarfärbebeispiele 20 – 29** (alle Mengenangaben in Gramm/100 g Färbemittel)

| Farbstoff / Beispiel Nr. | 20 | 21 | 22 | 23 | 24 | 25 | 26 | 27 | 28 |
|---|---|---|---|---|---|---|---|---|---|
| 2,4-Diamino-1-(2-methoxyethoxy)benzol* 2HCl | 1,6 | 1,4 | 0,02 | 0,8 | 0,6 | 0,1 | 0,6 | 0,6 | 0,3 |
| 2-Methoxymethyl-p-phenylendiamin* 2HCl | | 0,1 | | 1,0 | | | | | |
| 2-Methyl-p-phenylendiamin-sulfat | 0,1 | 1,0 | | | | | | | |
| 2-(2-Hydroxyethyl)-p-phenylendiamin-sulfat | | 0,1 | | | | | 1,0 | | |
| 2-Aminomethyl-p-phenylendiamin* 2HCl | | 0,05 | | | | 0,3 | | 1,5 | |
| 4-Amino-3-methyl-phenol | | 0,3 | | | | 0,3 | | | |
| 4,5-Diamino-1-((4-methylphenyl)methyl)-1H-pyrazol-sulfat | 1,5 | | | | | | 0,05 | | 0,2 |
| 4,5-Diamino-1-(2-hydroxyethyl)-1H-pyrazol-sulfat | 0,05 | | 0,1 | | 0,6 | | 0,3 | | |
| 4,5-Diamino-1-(1-methylethyl)-1H-pyrazol-sulfat | 0,03 | | | | | 0,02 | | | |
| 5-((2-Hydroxyethyl)amino)-2-methoxy-anilin-sulfat | 0,01 | | 0,02 | | | | | | |
| 1-(2-Hydroxyethylamino)-3,4-methylendioxybenzol | 0,2 | | | | | | | | |
| Resorcin | | 0,3 | | 0,2 | | | | 0,6 | |
| 2-Methylresorcin | 0,05 | 0,05 | | | | 0,1 | | 0,1 | |
| 5-Amino-2-methylphenol | 0,05 | 0,05 | | 0,2 | | | | | |
| 3-Amino-phenol | 0,3 | 0,06 | | 0,1 | | | 0,3 | 0,1 | |

**Tabelle 2:** (Fortsetzung)

| Farbstoff / Beispiel Nr. | 20 | 21 | 22 | 23 | 24 | 25 | 26 | 27 | 28 |
|---|---|---|---|---|---|---|---|---|---|
| 2-Amino-6-chlor-4-nitro-phenol | 0,1 | | 0,05 | | | | | 0,2 | |
| 2-Chlor-6-(ethylamino)-4-nitro-phenol | 0,1 | | 0,05 | | | | | 0,1 | |
| p-Phenylendiamin | | 0,5 | | | | 0,05 | | | |
| 2-(1-Hydroxyethyl)-p-phenylendiamin | | 0,02 | | 0,3 | | | | | |
| 2-(2,4-Diaminophenoxy)-ethanol-sulfat | | 0,05 | 0,05 | | 0,05 | | | | |
| N,N-Bis(2-hydroxyethyl)-p-phenylendiamin-sulfat | 0,1 | | | 0,2 | | 0,05 | | | 1,0 |
| 4-Aminophenol | | 0,02 | | | | | | | |
| 2,4-Diamino-1-fluor-5-methylbenzol-sulfat | | | | | 0,05 | | | | |
| 3-Amino-6-methoxy-2-(methylamino)pyridin * 2HCl | | 0,1 | | | | | | | |
| N-(3-(Dimethylamino)phenyl)-harnstoff | | | 0,05 | | 0,1 | | | 0,01 | |
| 5-Amino-6-chlor-o-kresol | | 0,1 | 0,05 | | 0,05 | 0,2 | 0,1 | | |
| 1-Naphthol | 0,08 | | | 0,1 | | 0,05 | | | 0,6 |
| 1-Acetoxy-2-methyl-naphthalin | 0,02 | | | | | | | | |
| 4-Chlorresorcin | | 0,1 | | | | | | 0,02 | |
| Sesamol | | 0,05 | | | | | | 0,02 | |

save

**Tabelle 2:** (Fortsetzung)

| **Farbstoff / Beispiel Nr.** | **20** | **21** | **22** | **23** | **24** | **25** | **26** | **27** | **28** |
|---|---|---|---|---|---|---|---|---|---|
| 6-Amino-m-kresol | 0,05 | | | 0,03 | | 0,05 | | | |
| 2,6-Diamino-3-pyridin-3-yl-azo-pyridin | 0,01 | | | | | | | 0,02 | |

**Tabelle 3:** **Färbeergebnisse der Beispiele 20 – 28**

| **Beispiel Nr.** | **Färbung** |
|---|---|
| **20** | braun-schwarz |
| **21** | blau-schwarz |
| **22** | rotbraun |
| **23** | blau-schwarz |
| **24** | granatrot |
| **25** | rotbraun |
| **26** | violettstichig schwarz |
| **27** | braun |
| **28** | violettstichig blau |

**Beispiel 29: Vergleichsversuch**

Zum Nachweis der besseren Säurestabilität/Schweißstabilität der mit den erfindungsgemäßen Haarfärbemittel erhaltenen Färbungen wurden Haare, die mit einem erfindungsgemäßen Mittel (A) beziehungsweise einem nicht-erfindungsgemäßen Mittel (B) gefärbt und anschließend mit einer Lösung von synthetischem Schweiß (mit Milchsäure auf pH=3,2 eingestellten Lösung von 10% Natriumchlorid, 1% Dikaliumhydrogenphosphat und 0,25% Histidin) 24 Stunden lang bei 37 °C behandelt. Die Haare wurden anschließend mit Wasser ausgespült und getrocknet. Der erhaltene Farbton wurde jeweils mit einem Farbmessgerät der Firma Minolta, Typ Chromameter II, im Lab-System gemessen und mit den entsprechenden Farbmesswerten der gefärbten Haare vor der Behandlung verglichen. Hierbei steht der L-Wert für die Helligkeit (das heißt je geringer der L-Wert ist, um so größer ist die Farbintensität), während der a-Wert ein Maß für den Rotanteil ist (das heißt je größer der a-Wert ist, um so größer ist der Rotanteil). Der b-Wert ist ein Maß für den Blauanteil der Farbe, wobei der Blauanteil um so größer ist, je negativer der b-Wert ist. Als Maß für die Stabilität der Färbung gegenüber Schweiß und Säuren wurde die Gesamtfarbänderung ΔE der Haarsträhnchen bei der Behandlung mit Säure nach folgender Formel ermittelt:

$$\Delta E = \sqrt{(L_0 - L_s)^2 + (a_0 - a_s)^2 + (b_0 - b_s)^2}$$

$L_0$: Helligkeit vor der Behandlung
$L_s$Helligkeit nach der Behandlung
$a_0$: Rot-Grün-Wert vor der Behandlung
$a_s$: Rot-Grün-Wert nach der Behandlung
$b_0$: Gelb-Blau-Wert vor der Behandlung
$b_s$: Gelb-Blau-Wert nach der Behandlung

**Haarfärbemittel A** (erfindungsgemäß)

| | |
|---|---|
| 10,0 g | Ethanol |
| 10,0 g | Natriumlaurylethersulfat, 28%ige Lösung in Wasser |
| 10,0 g | Ammoniak 25%ige wässrige Lösung |
| 0,3 g | Ascorbinsäure |
| 2,5 mmol | 2,4-Diamino-1-(2-methoxyethoxy)benzol -dihydrochlorid |
| 2,5 mmol | 4,5-Diamino-1-(2-hydroxyethyl)-1H-pyrazol-sulfat |
| ad 100,0 g | Wasser, entmineralisiert |

**Haarfärbemittel B** (nicht-erfindungsgemäß)

| | |
|---|---|
| 10,0 g | Ethanol |
| 10,0 g | Natriumlaurylethersulfat, 28%ige Lösung in Wasser |
| 10,0 g | Ammoniak 25%ige wässrige Lösung |
| 0,3 g | Ascorbinsäure |
| 2,5 mmol | 2-(2,4-Diaminophenoxy)-ethanol-dihydrochlorid |
| 2,5 mmol | 4,5-Diamino-1-(2-hydroxyethyl)-1 H-pyrazol-sulfat |
| ad 100,0 g | Wasser, entmineralisiert |

Tabelle 4:

| **Farbmesswerte** | | | | | | | |
|---|---|---|---|---|---|---|---|
| **Färbemittel** | **$L_0$** | **$a_0$** | **$b_0$** | **$L_s$** | **$a_s$** | **$b_s$** | **ΔE** |
| A | 21,4 | 23,6 | 0,2 | 30,0 | 26,9 | -0,7 | 9,3 |

Tabelle 4: (fortgesetzt)

| Farbmesswerte | | | | | | |
|---|---|---|---|---|---|---|
| Färbemittel | $L_0$ | $a_0$ | $b_0$ | $L_s$ | $a_s$ | $b_s$ | $\Delta E$ |
| B | 18,4 | 23,3 | 2,5 | 30,6 | 35,9 | 2,6 | 17,5 |

Alle Prozentangaben in der vorliegenden Anmeldung stellen, soweit nicht anders angegeben, Gewichtsprozente dar.

## Patentansprüche

**1.** Mittel zur Färbung von Keratinfasem auf der Basis einer Entwicklersubstanz-Kupplersubstanz-Kombination, **dadurch gekennzeichnet, dass** es die Kupplersubstanz 2,4-Diamino-1-(2-methoxy-ethoxy)benzol oder deren physiologisch verträgliches Salz und mindestens eine Entwicklersubstanz aus der Gruppe bestehend aus

(a) am Benzolring monosubstituierten p-Phenylendiaminderivaten der allgemeinen Formel (I) oder deren physiologisch verträglichen Salzen,

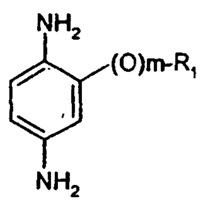

(I)

,

worin wenn **m** gleich 0 ist $\mathbf{R_1}$ einen geradkettigen oder verzweigten Monohydroxy-($C_1$-$C_6$)-alkylrest, einen geradkettigen oder verzweigten Polyhydroxy-($C_2$-$C_6$)-alkylrest, einen geradkettigen oder verzweigten Mono-($C_1$-$C_6$)-alkoxy-($C_1$-$C_6$)-alkylrest, einen geradkettigen oder verzweigten Poly-($C_1$-$C_6$)-alkoxy-($C_2$-$C_6$)-alkylrest, einen geradkettigen oder verzweigten Amino-($C_1$-$C_6$)-Alkylrest, oder eine carbozyklische oder heterozyklische, substituierte oder unsubstituierte, aromatische Verbindung darstellt; und wenn **m** gleich 1 ist $\mathbf{R_1}$ einen geradkettigen oder verzweigten $C_3$-$C_6$-Alkylrest, einen geradkettigen oder verzweigten Monohydroxy-($C_2$-$C_6$)-alkylrest, einen geradkettigen oder verzweigten Polyhydroxy-($C_3$-$C_6$)-alkylrest, einen geradkettigen oder verzweigten Mono-($C_1$-$C_6$)-alkoxy-($C_2$-$C_6$)-alkylrest, einen geradkettigen oder verzweigten Poly-($C_1$-$C_6$)-alkoxy-($C_3$-$C_6$)-alkylrest, einen geradkettigen oder verzweigten Amino-($C_2$-$C_6$)-Alkylrest, oder einen carbozyklische oder heterozyklische, substituierte oder unsubstituierte, aromatische Verbindung darstellt;
(b) 4,5-Diamino-1H-pyrazolderivaten der allgemeinen Formel (II) oder deren physiologisch verträglichen Salzen,

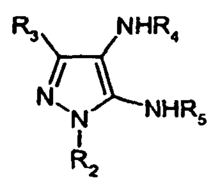

(II)

,

worin $\mathbf{R_2}$, $\mathbf{R_4}$ und $\mathbf{R_5}$ unabhängig voneinander ein Wasserstoffatom, einen geradkettigen oder verzweigten $C_1$-$C_6$-Alkylrest, einen geradkettigen oder verzweigten Monohydroxy-($C_1$-$C_6$)-alkylrest, einen geradkettigen oder verzweigten Polyhydroxy-($C_2$-$C_6$)-alkylrest, oder einen unsubstituierten oder am Aromaten substituierten Benzylrest darstellen,
und $\mathbf{R_3}$ gleich einem Wasserstoffatom, einem geradkettigen oder verzweigten $C_1$-$C_6$-Alkylrest, einem geradkettigen oder verzweigten Monohydroxy-($C_1$-$C_6$)-alkylrest, einem geradkettigen oder verzweigten Polyhydroxy-($C_2$-$C_6$)-alkylrest, einem geradkettigen oder verzweigten Mono-($C_1$-$C_6$)-alkoxy-($C_1$-$C_6$)-alkylrest, einem geradkettigen oder verzweigten Poly-($C_1$-$C_6$)-alkoxy-($C_2$-$C_6$)-alkylrest, oder einer carbozyklischen oder heterozyklischen, substituierten oder unsubstituierten, aromatischen Verbindung ist; und
(c) 4-Aminophenolderivaten der allgemeinen Formel (III) oder deren physiologisch verträglichen Salzen,

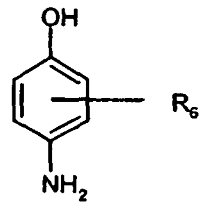
(III)

,

worin $R_6$ gleich einem geradkettigen oder verzweigten $C_1$-$C_6$-Alkylrest, einem geradkettigen oder verzweigten Monohydroxy-($C_1$-$C_6$)-alkylrest, einem geradkettigen oder verzweigten Polyhydroxy-($C_2$-$C_6$)-alkylrest, einem geradkettigen oder verzweigten Mono-($C_1$-$C_6$)-alkoxy-($C_1$-$C_6$)-alkylrest, einem geradkettigen oder verzweigten Poly-($C_1$-$C_6$)-alkoxy-($C_2$-$C_6$)-alkylrest, einem geradkettigen oder verzweigten Amino-($C_1$-$C_6$)-Alkylrest, oder einer carbozyklischen oder heterozyklischen, substituierten oder unsubstituierten, aromatischen Verbindung ist, unter der Bedingung, dass der Rest $R_6$ nicht gleich Methyl ist, wenn er in 2-Stellung steht; enthält.

2. Mittel nach Anspruch 1, **dadurch gekennzeichnet, dass** das p-Phenylendiaminderivat der Formel (I) ausgewählt ist aus 2-(Hydroxymethyl)-p-phenylendiamin, 2-(1-Hydroxyethyl)-p-phenylendiamin, 2-(2-Hydroxyethyl)-p-phenylendiamin, 2-(3-Hydroxypropyl)-p-phenylendiamin, 2-(1,2-Dihydroxyethyl)-p-phenylendiamin, 2-(Methoxymethyl)-p-phenylendiamin, 2-(2-Methoxyethyl)-p-phenylendiamin, 2-(2-Hydroxyethoxy)-p-phenylendiamin, 2,5-Diamino-1,1'-biphenyl, 2-(2-Thienyl)-p-phenylendiamin, 2-(3-Thienyl)-p-phenylendiamin und 3-(2,5-Diaminophenyl)pyridin.

3. Mittel nach Anspruch 1, **dadurch gekennzeichnet, dass** das Pyrazolderivat der Formel (II) ausgewählt ist aus 4,5-Diamino-1-methyl-1 H-pyrazol, 4,5-Diamino-1-(2-hydroxyethyl)-1H-pyrazol, 4,5-Diamino-1-(1-methylethyl)-1H-pyrazol, 4,5-Diamino-1-(phenylmethyl)-1H-pyrazol, 4,5-Diamino-1-((4-methylphenyl)methyl)-1H-pyrazol, 4,5-Diamino-1-(2-hydroxyethyl)-3-methyl-1H-pyrazol, 4,5-Diamino-1-methyl-3-phenyl-1H-pyrazol und 4,5-Diamino-1,3-dimethyl-1H-pyrazol.

4. Mittel nach Anspruch 1, **dadurch gekennzeichnet, daß** das p-Aminophenolderivat der Formel (III) ausgewählt ist aus 3-Methyl-4-amino-phenol, 2-Aminomethyl-4-aminophenol, 2-Hydroxymethyl-4-aminophenol, 3-Hydroxymethyl-4-aminophenol, 2-Phenyl-4-aminophenol, 2-(2-Thienyl)-4-aminophenol, 2-(3-Thienyl)-4-aminophenol, 3-(2-Thienyl)-4-aminophenol und 3-(3-Thienyl)-4-aminophenol.

5. Mittel nach einem der Ansprüche 1 bis 4, **dadurch gekennzeichnet, daß** es das 2,4-Diamino-1-(2-methoxyethoxy) benzol sowie die Verbindungen der Formel (I) bis (III) jeweils in einer Menge von 0,01 bis 5 Gewichtsprozent enthält.

6. Mittel nach einem der Ansprüche 1 bis 5, **dadurch gekennzeichnet, dass** es zusätzlich weitere Entwicklersubstanzen und/oder Kupplersubstanzen und/oder direktziehende Farbstoffe enthält.

7. Mittel nach einem der Ansprüche 1 bis 6, **dadurch gekennzeichnet, dass** es einen pH-Wert von 6,5 bis 11,5 aufweist.

8. Mittel nach einem der Ansprüche 1 bis 7, **dadurch gekennzeichnet, dass** es ein Haarfärbemittel ist.

9. Verfahren zum oxidativen Färben von Haaren, **dadurch gekennzeichnet, dass** man vor der Anwendung ein Haarfärbemittel nach einem der Ansprüche 1 bis 8 mit einem Oxidationsmittel vermischt, sodann auf das Haar aufträgt, bei einer Temperatur von 15 bis 50°C 10 bis 45 Minuten lang einwirken lässt, das Haar anschließend mit Wasser ausspült, gegebenenfalls shampooniert und sodann trocknet.

## Claims

1. Agent for colouring keratinic fibres based on a developer substance-coupler substance combination, **characterized in that** it comprises the coupler substance 2,4-diamino-1-(2-methoxyethoxy)benzene or its physiologically acceptable salt and at least one developer substance selected from the group consisting of

(a) p-phenylenediamine derivatives monosubstituted on the benzene ring and of the general formula (I) or their physiologically acceptable salts,

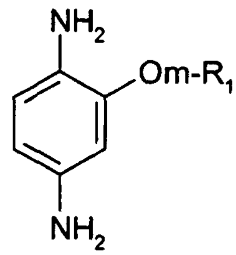

(I)

in which, when **m** is 0, $\mathbf{R_1}$ is a straight-chain or branched monohydroxy($C_1$-$C_6$)alkyl radical, a straight-chain or branched polyhydroxy-($C_2$-$C_6$)alkyl radical, a straight-chain or branched mono ($C_1$-$C_6$)alkoxy($C_1$-$C_6$)alkyl radical, a straight-chain or branched poly-($C_1$-$C_6$)alkoxy($C_2$-$C_6$)alkyl radical, a straight-chain or branched amino($C_1$-$C_6$)alkyl radical or a carbocyclic or heterocyclic, substituted or unsubstituted, aromatic compound; and when **m** is 1 $\mathbf{R_1}$ is a straight-chain or branched $C_3$-$C_6$alkyl radical, a straight-chain or branched monohydroxy ($C_2$-$C_6$)alkyl radical, a straight-chain or branched polyhydroxy-($C_3$-$C_6$)alkyl radical, a straight-chain or branched mono ($C_1$-$C_6$)alkoxy($C_2$-$C_6$)alkyl radical, a straight-chain or branched poly-($C_1$-$C_6$)alkoxy($C_3$-$C_6$) alkyl radical, a straight-chain or branched amino($C_2$-$C_6$)alkyl radical or a carbocyclic or heterocyclic, substituted or unsubstituted, aromatic compound;

(b) 4,5-diamino-1H-pyrazole derivatives of the general formula (II) or their physiologically acceptable salts,

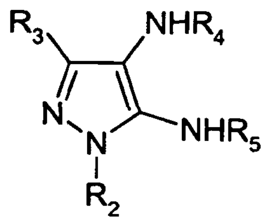

(II)

in which $\mathbf{R_2}$, $\mathbf{R_4}$ and $\mathbf{R_5}$, independently of one another, are a hydrogen atom, a straight-chain or branched $C_1$-$C_6$-alkyl radical, a straight-chain or branched monohydroxy-($C_1$-$C_6$)alkyl radical, a straight-chain or branched polyhydroxy($C_2$-$C_6$)alkyl radical, or a benzyl radical which is unsubstituted or substituted on the aromatic, and $\mathbf{R_3}$ is a hydrogen atom, a straight-chain or branched $C_1$-$C_6$alkyl radical, a straight-chain or branched monohydroxy($C_1$-$C_6$)alkyl radical, a straight-chain or branched polyhydroxy($C_2$-$C_6$)alkyl radical, a straight-chain or branched mono($C_1$-$C_6$)alkoxy($C_1$-$C_6$)alkyl radical, a straight-chain or branched poly-($C_1$-$C_6$) alkoxy($C_2$-$C_6$)alkyl radical, or a carbocyclic or heterocyclic, substituted or unsubstituted, aromatic compound; and

(c) 4-aminophenol derivatives of the general formula (III) or their physiologically acceptable salts,

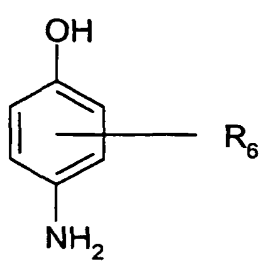

(III)

in which $\mathbf{R_6}$ is a straight-chain or branched $C_1$-$C_6$alkyl radical, a straight-chain or branched monohydroxy($C_1$-$C_6$) alkyl radical, a straight-chain or branched polyhydroxy-($C_2$-$C_6$)alkyl radical, a straight-chain or branched mono ($C_1$-$C_6$)alkoxy($C_1$-$C_6$)alkyl radical, a straight-chain or branched poly-($C_1$-$C_6$)alkoxy($C_2$-$C_6$)alkyl radical, a straight-chain or branched amino($C_1$-$C_6$)alkyl radical, or a carbocyclic or heterocyclic, substituted or unsubstituted, aromatic compound, under the condition that the radical $R_6$ is not methyl when it is in the 2-position.

2. Agent according to Claim 1, **characterized in that** the p-phenylenediamine derivative of the formula (I) is chosen from 2-(hydroxymethyl)-p-phenylenediamine, 2-(1-hydroxyethyl)-p-phenylenediamine, 2-(2-hydroxyethyl)-p-phenylenediamine, 2-(3-hydroxypropyl)-p-phenylenediamine, 2-(1,2-dihydroxyethyl)-p-phenylenediamine, 2-(methoxymethyl)-p-phenylenediamine, 2-(2-methoxyethyl)-p-phenylenediamine, 2-(2-hydroxyethoxy)-p-phenylenediamine, 2,5-diamino-1,1'-biphenyl, 2-(2-thienyl)-p-phenylenediamine, 2-(3-thienyl)-p-phenylenediamine and 3-(2,5-diaminophenyl)pyridine.

3. Agent according to Claim 1, **characterized in that** the pyrazole derivative of the formula (II) is chosen from 4,5-diamino-1-methyl-1H-pyrazole, 4,5-diamino-1-(2-hydroxyethyl)-1H-pyrazole, 4,5-diamino-1-(1-methylethyl)-1H-pyrazole, 4,5-diamino-1-(phenylmethyl)-1H-pyrazole, 4,5-diamino-1-((4-methylphenyl)methyl)-1H-pyrazole, 4,5-diamino-1-(2-hydroxyethyl)-3-methyl-1H-pyrazole, 4,5-diamino-1-methyl-3-phenyl-1H-pyrazole and 4,5-diamino-1,3-dimethyl-1H-pyrazole.

4. Agent according to Claim 1, **characterized in that** the p-aminophenol derivative of the formula (III) is chosen from 3-methyl-4-aminophenol, 2-aminomethyl-4-aminophenol, 2-hydroxymethyl-4-aminophenol, 3-hydroxymethyl-4-aminophenol, 2-phenyl-4-aminophenol, 2-(2-thienyl)-4-aminophenol, 2-(3-thienyl)-4-aminophenol, 3-(2-thienyl)-4-aminophenol and 3-(3-thienyl)-4-aminophenol.

5. Agent according to one of Claims 1 to 4, **characterized in that** it comprises the 2,4-diamino-1-(2-methoxyethoxy) benzene and the compounds of the formula (I) to (III) in each case in an amount of from 0.01 to 5 per cent by weight.

6. Agent according to one of Claims 1 to 5, **characterized in that** it additionally comprises further developer substances and/or coupler substances and/or direct dyes.

7. Agent according to one of Claims 1 to 6, **characterized in that** it has a pH of from 6.5 to 11.5.

8. Agent according to one of Claims 1 to 7, **characterized in that** it is a hair colorant.

9. Method for the oxidative colouring of hair, **characterized in that**, prior to application, a hair colorant according to one of Claims 1 to 8 is mixed with an oxidizing agent, then applied to the hair, left to act at a temperature of from 15 to 50°C for 10 to 45 minutes, the hair is then rinsed with water, optionally shampooed and then dried.

## Revendications

1. Composition pour la teinture de fibres de kératine, à base d'une association développeur-coupleur, **caractérisée en ce qu'**elle contient le coupleur 2,4-diamino-1-(2-méthoxy-éthoxy)benzène ou un sel physiologiquement acceptable de celui-ci et au moins un développeur choisi dans le groupe constitué par

   (a) des dérivés de p-phénylènediamine monosubstitués sur le noyau benzénique, de formule générale (I) ou leurs sels physiologiquement acceptables,

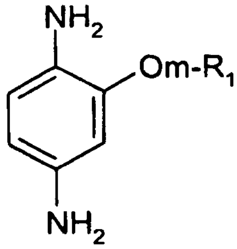

(I)

   formule dans laquelle lorsque **m** est égal à 0 $R_1$ représente un radical monohydroxyalkyle en $C_1$-$C_6$ à chaîne droite ou ramifiée, un radical polyhydroxyalkyle en $C_2$-$C_6$, à chaîne droite ou ramifiée, un radical monoalcoxy($C_1$-$C_6$)-alkyle($C_1$-$C_6$) à chaîne droite ou ramifiée, un radical polyalcoxy($C_1$-$C_6$)-alkyle($C_2$-$C_6$) à chaîne droite

ou ramifiée, un radical aminoalkyle($C_1$-$C_6$) à chaîne droite ou ramifiée, ou un composé aromatique carbocyclique ou hétérocyclique, substitué ou non substitué ; et lorsque **m** est égal à 1 $\mathbf{R_1}$ représente un radical alkyle en $C_3$-$C_6$ à chaîne droite ou ramifiée, un radical monohydroxyalkyle en $C_2$-$C_6$ à chaîne droite ou ramifiée, un radical polyhydroxyalkyle en $C_3$-$C_6$ à chaîne droite ou ramifiée, un radical monoalcoxy($C_1$-$C_6$)-alkyle($C_2$-$C_6$) à chaîne droite ou ramifiée, un radical polyalcoxy($C_1$-$C_6$)-alkyle-($C_3$-$C_6$) à chaîne droite ou ramifiée, un radical aminoalkyle($C_2$-$C_6$) à chaîne droite ou ramifiée, ou un composé aromatique carbocyclique ou hétérocyclique, substitué ou non substitué ;

(b) des dérivés de 4,5-diamino-1H-pyrazole de formule générale (II) ou leurs sels physiologiquement acceptables,

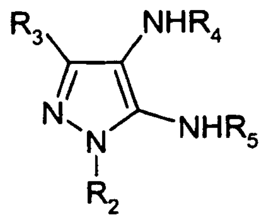

(II)

formule dans laquelle $\mathbf{R_2}$, $\mathbf{R_4}$ et $\mathbf{R_5}$ représentent, indépendamment les uns des autres, un atome d'hydrogène, un radical alkyle en $C_1$-$C_6$ à chaîne droite ou ramifiée, un radical monohydroxyalkyle en $C_1$-$C_6$ à chaîne droite ou ramifiée, un radical polyhydroxyalkyle en $C_2$-$C_6$ à chaîne droite ou ramifiée, ou un radical benzyle substitué ou non substitué sur le noyau aromatique,

et $\mathbf{R_3}$ représente un atome d'hydrogène, un radical alkyle en $C_1$-$C_6$ à chaîne droite ou ramifiée, un radical monohydroxyalkyle en $C_1$-$C_6$ à chaîne droite ou ramifiée, un radical polyhydroxyalkyle en $C_2$-$C_6$ à chaîne droite ou ramifiée, un radical monoalcoxy($C_1$-$C_6$)-alkyle($C_1$-$C_6$) à chaîne droite ou ramifiée, un radical polyalcoxy($C_1$-$C_6$)-alkyle-($C_2$-$C_6$) à chaîne droite ou ramifiée, ou un composé aromatique carbocyclique ou hétérocyclique, substitué ou non substitué ; et

c) des dérivés de 4-aminophénol de formule générale (III) ou leurs sels physiologiquement acceptables,

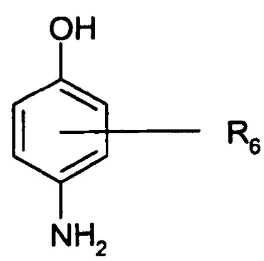

(III)

formule dans laquelle $\mathbf{R_6}$ représente un radical alkyle en $C_1$-$C_6$ à chaîne droite ou ramifiée, un radical monohydroxyalkyle en $C_1$-$C_6$ à chaîne droite ou ramifiée, un radical polyhydroxyalkyle en $C_2$-$C_6$ à chaîne droite ou ramifiée, un radical monoalcoxy ($C_1$-$C_6$)-alkyle($C_1$-$C_6$) à chaîne droite ou ramifiée, un radical polyalcoxy ($C_1$-$C_6$)-alkyle-($C_2$-$C_6$) à chaîne droite ou ramifiée, un radical aminoalkyle($C_1$-$C_6$) à chaîne droite ou ramifiée, ou un composé aromatique carbocyclique ou hétérocyclique, substitué ou non substitué, étant entendu que le radical $\mathbf{R_6}$ ne représente pas le groupe méthyle lorsqu'il se trouve en position 2.

**2.** Composition selon la revendication 1, **caractérisée en ce que** le dérivé de p-phénylènediamine de formule (I) est choisi parmi la 2-(hydroxyméthyl)-p-phénylènediamine, la 2-(1-hydroxyéthyl)-p-phénylènediamine, la 2-(2-hydroxyéthyl)-p-phénylènediamine, la 2-(3-hydroxypropyl)-p-phénylènediamine, la 2-(1,2-dihydroxyéthyl)-p-phénylènediamine, la 2-(méthoxyméthyl)-p-phénylènediamine, la 2-(2-méthoxyéthyl)-p-phénylènediamine, la 2-(2-hydroxyéthoxy)-p-phénylènediamine, le 2,5-diamino-1,1'-biphényle, la 2-(2-thiényl)-p-phénylènediamine, la 2-(3-thiényl)-p-phénylènediamine et la 3-(2,5-diaminophényl)pyridine.

**3.** Composition selon la revendication 1, **caractérisée en ce que** le dérivé de pyrazole de formule (II) est choisi parmi le 4,5-diamino-1-méthyl-1H-pyrazole, le 4,5-diamino-1-(2-hydroxyéthyl)-1H-pyrazole, le 4,5-diamino-1-(1-méthyléthyl)-1H-pyrazole, le 4,5-diamino-1-(phénylméthyl)-1H-pyrazole, le 4,5-diamino-1-((4-méthylphényl)-méthyl)-1H-pyrazole, le 4,5-diamino-1-(2-hydroxyéthyl)-3-méthyl-1H-pyrazole, le 4,5-diamino-1-méthyl-3-phényl-1H-pyrazole et le 4,5-diamino-1,3-diméthyl-1H-pyrazole.

4. Composition selon la revendication 1, **caractérisée en ce que** le dérivé de p-aminophénol de formule (III) est choisi parmi le 3-méthyl-4-aminophénol, le 2-aminométhyl-4-aminophénol, le 2-hydroxyméthyl-4-aminophénol, le 3-hydroxyméthyl-4-aminophénol, le 2-phényl-4-aminophénol, le 2-(2-thiényl)-4-aminophénol, le 2-(3-thiényl)-4-aminophénol, le 3-(2-thiényl)-4-aminophénol et le 3-(3-thiényl)-4-aminophénol.

5. Composition selon l'une quelconque des revendications 1 à 4, **caractérisée en ce qu'**elle contient le 2,4-diamino-1-(2-méthoxyéthoxy)benzène ainsi que les composés de formules (I) à (III), chacun en une quantité de 0,01 à 5 % en poids.

6. Composition selon l'une quelconque des revendications 1 à 5, **caractérisée en ce qu'**elle contient additionnellement d'autres développeurs et/ou coupleurs et/ou colorants directs.

7. Composition selon l'une quelconque des revendications 1 à 6, **caractérisée en ce qu'**elle présente un pH de 6,5 à 11,5.

8. Composition selon l'une quelconque des revendications 1 à 7, **caractérisée en ce qu'**il s'agit d'une composition de teinture pour cheveux.

9. Procédé pour la teinture des cheveux par oxydation, **caractérisé en ce qu'**avant l'emploi on mélange avec un oxydant une composition de teinture selon l'une quelconque des revendications 1 à 8, puis on l'applique sur les cheveux, on laisse agir pendant 10 à 45 minutes à une température de 15 à 50°C, puis on rince les cheveux à l'eau, éventuellement on les shampooine et ensuite on les sèche.